# FASCICULE DE BREVET EUROPEEN

(11) **EP 3 031 437 B1**
(45) Date de publication et mention de la délivrance du brevet: **13.09.2017**
(21) Numéro de dépôt: 15197811.1
(22) Date de dépôt: 03.12.2015
(51) Int. Cl.: A61H 23/00, A61H 39/00, A61B 5/04, A61N 1/36, A61M 21/00, A61B 5/08, A61B 5/145

(54) **DISPOSITIF D'OPTIMISATION D'UN TRAITEMENT DU SYNDROME D'APNÉE DU SOMMEIL PAR STIMULATION KINESTHÉSIQUE**
VORRICHTUNG ZUR OPTIMIERUNG DER BEHANDLUNG DES SCHLAFAPNOE-SYNDROMS DURCH KINÄSTHETISCHE STIMULATION
DEVICE FOR OPTIMISING TREATMENT OF SLEEP APNOEA SYNDROME BY KINESTHETIC STIMULATION

(30) Priorité: 08.12.2014 FR 1462054
(43) Date de publication de la demande: 15.06.2016
(73) Titulaire: Sorin CRM SAS, 92140 Clamart Cedex (FR); INSERM - Institut National de la Santé et de la Recherche Médicale, 75013 Paris (FR); Université Grenoble Alpes, 38400 Saint-Martin-d'Hères (FR); Université de Rennes 1, 35065 Rennes Cedex (FR)
(72) Inventeur: GRAINDORGE, Laurence, 44470 Thouaré sur Loire (FR); AMBLARD, Amel, 92330 SCEAUX (FR); FEUERSTEIN, Delphine, 92130 Issy les Moulineaux (FR); HERNANDEZ, Alfredo, 35510 Cesson Sévigné (FR); PEPIN, Jean-Louis, 38000 Grenoble (FR)
(74) Mandataire: Grünecker Patent- und Rechtsanwälte PartG mbB

(56) Documents cités:
- US-A- 5 555 891
- US-A1- 2010 048 985
- US-A1- 2013 144 152
- US-A1- 2014 051 938

## Description

L'invention concerne le diagnostic et le traitement des troubles du sommeil.

Elle concerne plus particulièrement l'utilisation à cet effet d'un dispositif dit de "stimulation kinesthésique", c'est-à-dire d'un dispositif de stimulation sensorielle externe du patient au moyen d'un vibreur en contact avec la peau dans une région sensible, précise du corps de ce patient. L'activation de ce vibreur a pour effet d'exciter localement les terminaisons cutanées ou mécano-récepteurs de la peau, et de déclencher une réponse du système nerveux autonome du patient, à prépondérance sympathique (ci-après "réponse autonomique").

La réponse autonomique à une activation sympathique est observable sur les principaux effets modulateurs de l'activité cardiaque, par exemple :
- effet chronotrope : augmentation de la fréquence cardiaque, ou diminution des intervalles RR ;
- effet inotrope : augmentation de la contractilité cardiaque.

Cette réponse autonomique est également observable sur la vasoconstriction périphérique, qui est augmentée en cas d'activation autonomique sympathique.

Outre ces conséquences sur l'activité cardiaque, une activation sympathique provoque des réponses sur le système respiratoire ou sur le système nerveux central (réveils autonomiques).

Une telle technique, non invasive, permet d'agir sur un certain nombre de troubles respiratoires en alternative aux approches thérapeutiques classiques basées sur l'application d'une pression positive continue à travers un masque facial (traitement dit par PPC), sur l'utilisation d'une orthèse de propulsion mandibulaire et/ou sur la stimulation électrique du nerf grand hypoglosse, ce qui implique la pose d'un implant de type stimulateur.

En particulier, la pathologie respiratoire connue sous le nom de "syndrome d'apnée du sommeil" (SAS) est caractérisée par la survenue fréquente (au moins 10 à 20 fois par heure) d'apnées pendant une phase de sommeil du patient, une "apnée" (ou pause respiratoire) étant définie comme un arrêt temporaire de la fonction respiratoire de durée supérieure à 10 secondes. Elle peut également être caractérisée par la survenue d'hypopnées dans les mêmes conditions, une "hypopnée" étant définie comme une décroissance importante (mais sans interruption) du débit respiratoire, typiquement une décroissance de plus de 50 % par rapport à une moyenne de référence antérieure. Dans la suite de la description, on ne distinguera pas ces deux phénomènes, une référence à des "apnées" étant censée inclure également les hypopnées.

Cette pathologie atteint plus de 4 % de la population et plus de 50 % des patients souffrant d'insuffisance cardiaque. Afin de protéger l'individu contre l'asphyxie liée à la diminution de la concentration en oxygène du sang pendant l'interruption ou la réduction du débit respiratoire, le corps s'adapte mais avec un effet délétère sur le sommeil, entrainant des micro-réveils inconscients. Il s'ensuit en phase d'éveil une somnolence diurne avec perte d'attention et un accroissement des risques d'accident de la route. Par ailleurs, plusieurs études ont montré chez les patients souffrant de SAS une plus grande incidence de troubles tels qu'hypertension artérielle, arythmies ventriculaires, infarctus myocardique et insuffisance cardiaque.

Plusieurs documents décrivent la possibilité de stopper les épisodes d'apnée grâce à un traitement par stimulation.

Le US 5 555 891 A décrit ainsi un système de stimulation vibrotactile pour arrêter les apnées chez les nouveau-nés. L'objectif est de fournir un système capable de détecter les apnées et de stimuler l'enfant pour arrêter l'apnée, avec une énergie de stimulation qui peut varier pour éviter l'habituation. L'énergie appliquée est importante, ce qui entrainera souvent un réveil.

Le WO 2007/141345 A1 décrit un système de *télémonitoring* pour les services de néonatologie, permettant de détecter et de stimuler les apnées-bradycardies du nourrisson. Cette demande mentionne un ajustement de l'énergie de stimulation en fonction de la fréquence cardiaque mesurée du nourrisson. L'initialisation des paramètres de stimulation est effectuée à partir d'une base de données préétablie et, de ce fait, ne tient pas compte des spécificités physiologiques de l'individu à traiter (poids, sexe, etc.), alors que des études cliniques ont montré une dépendance de la réponse à la stimulation kinesthésique en fonction de ces paramètres physiologiques.

Le WO 96/28093 A1 enseigne également un système qui délivre un stimulus pour réduire la fréquence ou la durée d'un épisode d'apnée. Au niveau de la stimulation, ce document décrit simplement des limites basse et haute de l'énergie de stimulation pour espérer arrêter l'apnée sans réveiller le patient.

Le WO 2009/154458 A2 enseigne un système qui détecte les apnées et en retour provoque un réflexe d'inspiration, grâce à une stimulation dans la région de l'oreille. Divers moyens de détection des apnées sont cités, et la stimulation peut être électrique ou mécanique. La stratégie de stimulation est simple, et consiste à appliquer des trains de stimulations aussi longtemps que le trouble est présent. Les paramètres de stimulation sont indiqués comme pouvant varier, sans indiquer toutefois de règles de variation. La variation des paramètres de stimulation pour éviter l'habituation est également citée.

Enfin le US 2008/009915 A1 décrit un système qui détecte les troubles respiratoires à l'aide d'une canule nasale ou autre et qui applique une stimulation notamment vibratoire dans la région de l'oreille. L'objectif est d'arrêter l'apnée sans réveiller le patient, par une stimulation qui peut-être modulée, manuellement ou automatiquement, en fonction de caractéristiques physiologiques du patient ou des cycles du sommeil. Ce document cite également d'une façon générale l'optimisation des paramètres de stimulation pour s'adapter à la gravité des troubles du patient, sans toutefois donner de détail sur leur ajustement. La variation des paramètres de stimulation pour éviter l'habituation est également citée.

Le US 2010/0048985 A1 décrit un dispositif comparable, permettant d'appliquer des stimuli par des moyens de natures diverses (stimulation sonore ou ultrasonore de l'oreille, stimulation oculaire, agitateur mécanique, etc.). Le dispositif comprend également des moyens d'analyse de l'activité respiratoire permettant d'évaluer l'efficacité de la stimulation afin que le patient ou le médecin puissent modifier si besoin le paramétrage du générateur avec un dosage différent des stimuli.

La présente invention propose un système qui optimise les paramètres de stimulation à la fois en prévision de troubles respiratoires à venir mais également au cours de la survenue d'un trouble, de façon à ajuster l'énergie de stimulation à un niveau adapté au trouble et à son éventuelle persistance, tout en minimisant les risques de réveil du patient.

Plus précisément, l'invention propose un dispositif de traitement d'un trouble respiratoire chez un patient par stimulation kinesthésique, comprenant, comme divulgué par exemple par le US 2010/0048985 A1 précité :
- des moyens de détection d'un trouble respiratoire ;
- des moyens de commande aptes à produire des signaux de commande de stimulation kinesthésique en réponse à une détection d'un trouble respiratoire ;
- au moins un effecteur kinesthésique apte à être appliqué sur un site cutané externe du patient, et comprenant un transducteur électromécanique vibrant apte à recevoir les signaux de commande et à délivrer une énergie de stimulation kinesthésique déterminée par lesdits signaux ;
- des moyens pour déterminer l'efficacité d'une stimulation, aptes à détecter une disparition dudit trouble respiratoire ; et
- des moyens de détermination d'une énergie de stimulation.
Les moyens de détermination d'une énergie de stimulation sont des moyens aptes à :
· sélectionner une valeur d'énergie de départ et à faire varier la valeur d'énergie en fonction de l'efficacité d'une stimulation précédente, ladite valeur d'énergie étant plafonnée à une valeur maximale, et
· pour une première stimulation, sélectionner une valeur initiale en fonction de paramètres physiologiques du patient.

Selon diverses caractéristiques avantageuses :
- les moyens de détermination d'une énergie de stimulation comprennent des moyens aptes, pour une stimulation faisant suite à une stimulation précédente, à déterminer la valeur d'énergie de départ en fonction d'une valeur d'énergie efficace ayant fait cesser le trouble respiratoire lors d'une stimulation précédente ;
- la valeur maximale est une valeur variable fonction d'au moins un facteur du groupe formé par : sexe, âge, indice IMC et stade de sommeil du patient, ainsi que le type de trouble respiratoire dont est affecté le patient ;
- les moyens de détermination d'une énergie de stimulation comprennent des moyens aptes à relever le niveau d'énergie de façon incrémentale à partir de la valeur d'énergie de départ, aussi longtemps qu'un trouble respiratoire intervenu ne disparait pas ;
- les moyens de détermination d'une énergie de stimulation comprennent des moyens aptes à comparer chaque valeur d'énergie incrémentée à la valeur maximale ;
- les moyens de détermination d'une énergie de stimulation comprennent des moyens aptes à fixer une valeur d'énergie supérieure à la valeur maximale admissible en cas de détection d'un épisode grave de trouble respiratoire ;
- les moyens de détermination d'une énergie de stimulation comprennent des moyens aptes à relever la valeur maximale admissible d'énergie à la suite d'un dépassement de la valeur maximale admissible lors d'un trouble respiratoire précédent, sans réveil du patient ;
- les moyens de détermination d'une énergie de stimulation comprennent des moyens aptes à sélectionner la valeur d'énergie initiale à partir d'au moins une table de valeurs d'énergie initiale pour différentes combinaisons de sexe, d'âge, de taille et de poids et/ou en fonction d'un type de trouble respiratoire détecté ou paramétré.

On va maintenant décrire un exemple de mise en oeuvre de la présente invention, en référence aux dessins annexés où les mêmes références désignent d'une figure à l'autre des éléments identiques ou fonctionnellement semblables.
La Figure 1 illustre schématiquement un système selon l'invention, équipant un patient.
La Figure 2 illustre un tableau de valeurs d'énergie de stimulation initiale mémorisé dans le système.
La Figure 3 est un organigramme général d'un procédé d'ajustement de l'énergie de stimulation mis en oeuvre par le système.
Les Figures 3A à 3D illustrent quatre organigrammes particuliers associés à l'organigramme général de la Figure 3.

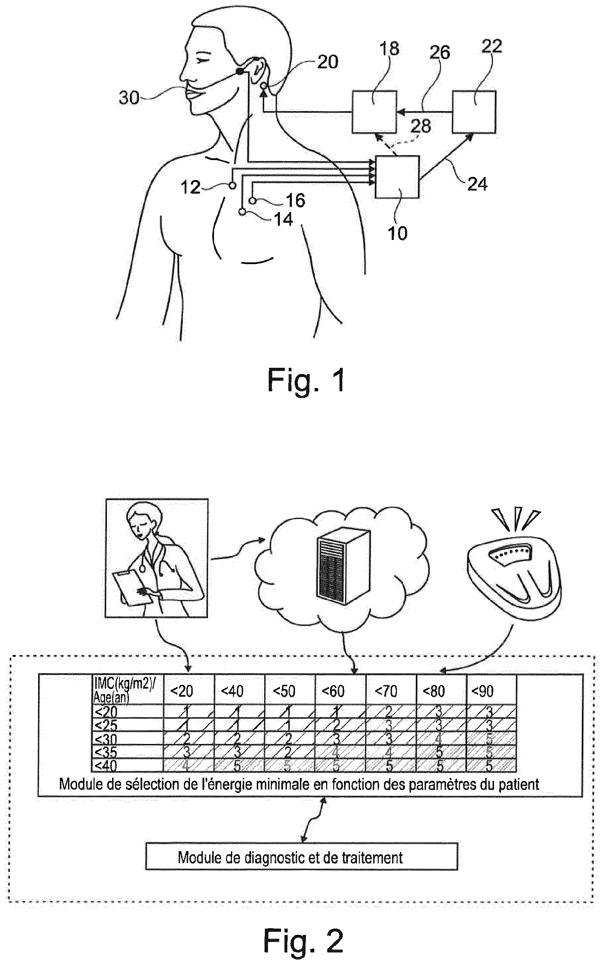

La Figure 1 illustre schématiquement les principaux composants d'un système utilisable pour la mise en oeuvre de la présente invention.

Ce système comporte un enregistreur Holter 10 relié à divers capteurs ou électrodes 12, 14, 16, permettant de mesurer des signaux physiologiques tels que la fréquence cardiaque, la respiration, la saturation en oxygène, l'onde de pouls, le phonocardiogramme, etc. Dans la suite, on s'intéressera principalement à l'amplitude ou à la pression respiratoire, qui est un paramètre simple à obtenir, mais cette mesure n'est pas limitative et l'invention peut être mise en oeuvre à partir d'autres signaux physiologiques recueillis sur le patient.

Le système comprend par ailleurs un dispositif de stimulation kinesthésique, avec un boitier générateur 18 produisant des impulsions de commande d'un effecteur de stimulation kinesthésique 20, constitué par exemple d'un vibreur placé dans une région sensible de l'épiderme, typiquement (chez l'adulte) dans la région de l'os mastoïde au voisinage de l'oreille. La stimulation vibrotactile appliquée sur la peau par l'effecteur 20 est détectée par les récepteurs sensoriels ou mécanorécepteurs de l'organisme, et cette information est ensuite transmise par l'intermédiaire des nerfs sensitifs au système nerveux autonome.

L'effecteur 20 est par exemple un transducteur du type *C10-100* de Precision Microdrives ou *C2 Tactor* de Engineering Acoustics. Il s'agit d'un transducteur de quelques grammes susceptible d'émettre des vibrations grâce à un vibreur intégré excité par des trains d'impulsions d'amplitude et de durée variables, la fréquence de ces impulsions étant typiquement proche de celle à laquelle les mécanorécepteurs de la peau sont les plus sensibles. D'autres types d'effecteur peuvent bien entendu être efficacement utilisés.

Le boitier de commande 18 est piloté par un microcontrôleur et possède des moyens permettant de régler l'intensité (c'est-à-dire l'énergie) de la stimulation kinesthésique, par variation contrôlée de l'amplitude et/ou du nombre, de la durée et/ou de la fréquence de stimulation des trains d'impulsions formant le signal appliqué à l'effecteur 20.

Le système comprend également un boitier 22 couplé au dispositif Holter 10 et au boitier de commande 18 par une liaison filaire ou sans fil respective 24, 26, de manière à recevoir des données du dispositif Holter 10, traiter ces données et produire en réponse des informations de contrôle de la stimulation kinesthésique qui seront transmises au boitier de commande 18. En variante, le traitement des données et le pilotage du boitier de commande 18 peuvent être opérés au sein même du dispositif Holter 10 et transmis directement par une liaison 28 au boitier 18.

Le système comprend enfin des moyens de mesure du débit respiratoire, par exemple une canule de pression nasale 30 (et/ou une canule buccale) ou autre type de capteur tel que thermistance ou capteur mécanique des variations de volume de l'abdomen et/ou de la cage thoracique (au moyen d'une ceinture équipée de capteurs sensibles à l'étirement). Ces moyens délivrent au dispositif Holter 10 un signal ventilatoire analysé en continu de manière à détecter en temps réel la survenue d'une apnée ou d'une hypopnée.

Le fonctionnement du système est le suivant : lorsqu'une apnée est détectée par le dispositif Holter 10, le boitier de commande 18 déclenche un stimulus kinesthésique propre à déclencher une réponse du système nerveux autonome de sorte que, en réponse, le système nerveux autonome va provoquer une variation de la respiration et donc enclencher un réflexe respiratoire qui endiguera l'apnée.

L'énergie avec laquelle est effectuée cette stimulation est primordiale. Ainsi :
- si elle est trop faible, elle ne sera pas efficace et ne produira aucun effet ;
- si elle est trop élevée, la stimulation risque de réveiller le patient ;
- si elle est trop répétitive, un phénomène d'habituation peut se créer, qui peut à son tour diminuer l'efficacité de la stimulation.

En outre, la même énergie peut avoir une efficacité différente en fonction du patient, du stade de sommeil ou même de l'évènement apnéique lui-même.

Le système tel que décrit en référence à la Figure 1 est apte à optimiser en temps réel l'énergie de la stimulation kinesthésique afin de pallier les problèmes ci-dessus. On comprendra que pour moduler l'"énergie" de stimulation, on peut agir aussi bien sur l'intensité des vibrations de stimulation que sur leur fréquence d'apparition, leur nombre, leur forme d'enveloppe, etc. ou toute combinaison de ces paramètres.

Cette optimisation de l'énergie est réalisée tout d'abord grâce à une initialisation de départ en fonction des paramètres du patient. Cette initialisation permet de fixer l'énergie initiale de la stimulation.

Les inventeurs ont ainsi déterminé que l'efficacité d'une même énergie délivrée dans les mêmes conditions (épisode d'apnée identique, état de sommeil identique) est susceptible de changer en fonction des caractéristiques physiques du patient telles que le sexe, l'âge et l'indice de masse corporelle (IMC). Ainsi, plus le sujet est âgé, plus l'énergie doit être grande pour obtenir la même efficacité. De même, lorsque l'IMC augmente, l'énergie doit être également augmentée.

Le système est ensuite apte à adapter l'énergie nécessaire au traitement au cours de la nuit et le cas échéant au cours d'un même épisode en fonction de l'efficacité de la stimulation et du risque d'éveil.

Selon une mise en oeuvre particulière, et en référence à la Figure 2, une table d'énergie de défaut en fonction de ces trois paramètres (sexe, âge et IMC - la Figure 2 montrant une table pour l'un des deux sexes seulement) est établie et mémorisée en fonction des données cliniques existantes dans le domaine. Pour cela, une population de patients sujets à apnées font l'objet d'une stimulation lors d'un enregistrement polysomno-graphique avec des énergies de stimulation variables, par exemple variant aléatoirement. L'efficacité de la stimulation est mesurée et l'analyse de la population permet de déterminer les énergies efficaces minimales en fonction des caractères cliniques des patients.

Lors de la mise en route du système de stimulation, ces données sont demandées à l'utilisateur et une valeur d'énergie par défaut est alors mémorisée comme valeur initiale en fonction de ces données.

La possibilité de mettre à jour ces données est avantageusement prévue pour le cas où intervient par exemple une importante chute ou prise de poids du patient, ce facteur pouvant modifier l'efficacité de la thérapie si le système ne modifie pas l'énergie en conséquence.

La Figure 2 illustre, outre le tableau de valeurs, les modalités de détermination de l'énergie de départ. Le sexe, la valeur de l'IMC et l'âge sont saisis dans le système à l'aide d'une interface utilisateur (écran tactile, combinaison clavier-écran, etc.) soit directement par le médecin sur le système lors d'une consultation, soit toujours par le médecin via une liaison réseau, soit via le patient lui-même (par exemple à l'aide d'une balance reliée par réseau de proximité tel que Bluetooth au système).

Le système utilise alors le tableau de données précité, stocké dans sa mémoire, pour déterminer l'énergie de départ appropriée en fonction de ces paramètres.

À partir de cette initialisation, le système est apte à faire varier l'énergie de stimulation pour maintenir une énergie efficace mais sans provoquer un réveil du patient.

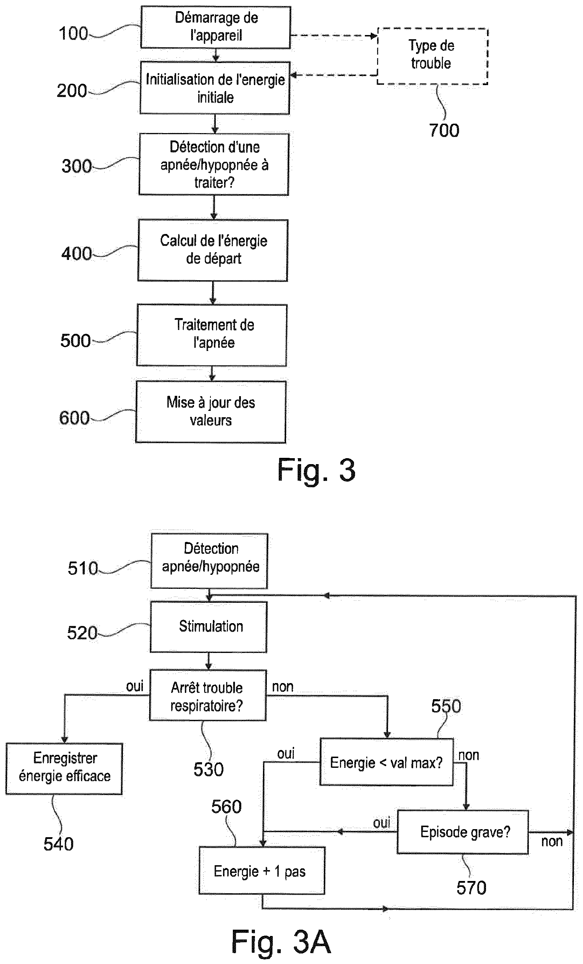

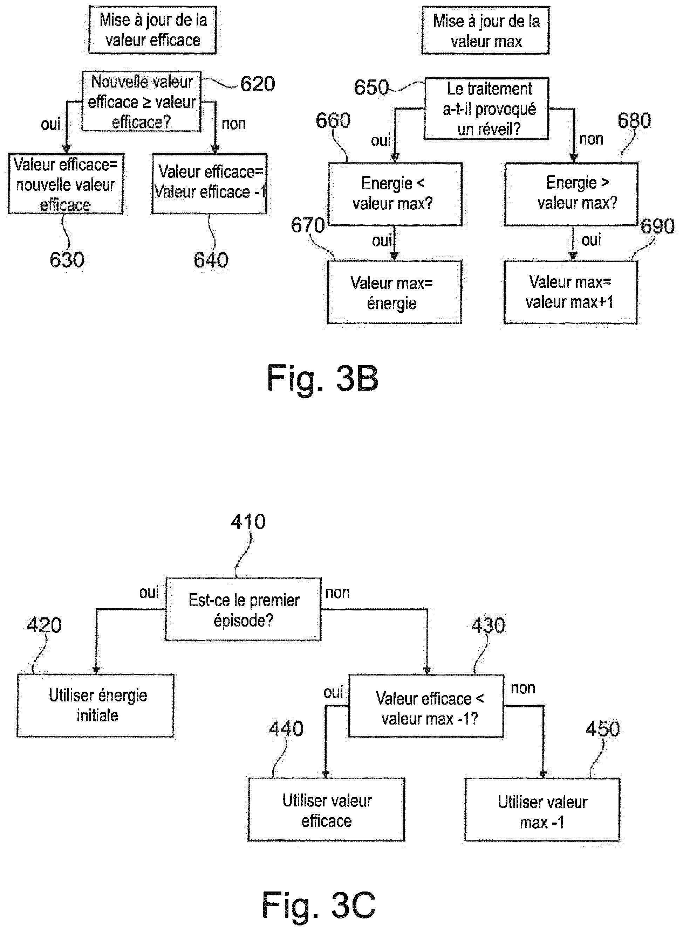

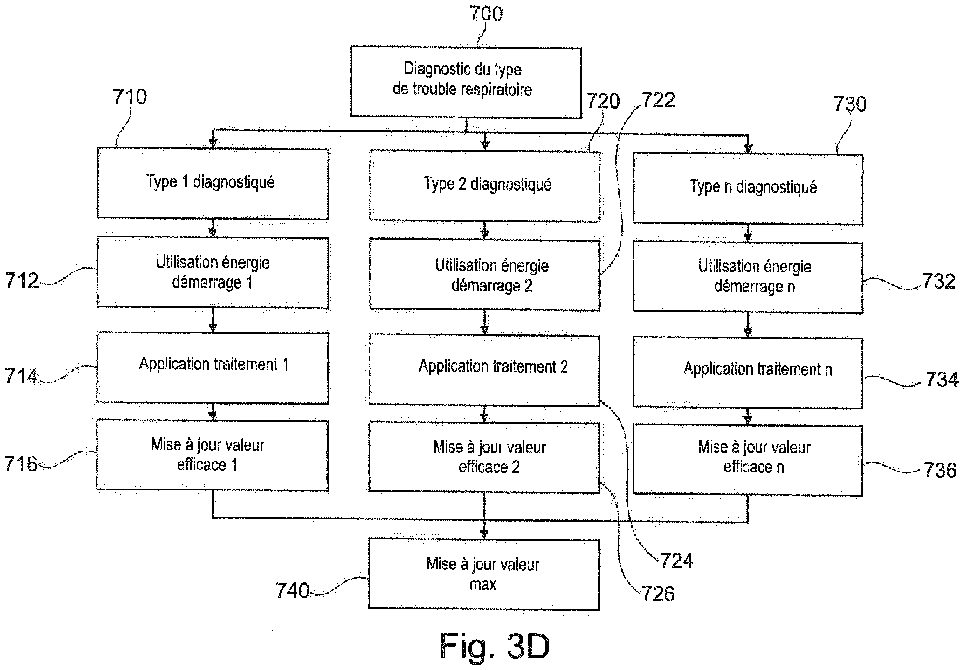

Ainsi, en référence à la Figure 3, après la mise en marche de l'appareil (étape 100) l'initialisation de la valeur d'énergie de stimulation décrite plus haut est mise en oeuvre (étape 200).

Lorsqu'un évènement à traiter survient (détection d'apnée ou d'hypopnée à l'étape 300), un algorithme exécuté dans l'unité de commande du système détermine d'abord l'énergie qui sera utilisée au démarrage du traitement (étape 400, détaillée à la Figure 4C). Ensuite, au cours de la phase de traitement (étape 500, détaillée à la Figure 3A), l'énergie peut évoluer en fonction de l'efficacité observée et d'une limite maximale liée au risque de réveil. Une fois l'évènement terminé, le système est apte à optimiser les énergies à utiliser pendant la phase de mise à jour des valeurs (étape 600, détaillée à la Figure 3B).

Les détails de ce processus sont illustrés sur les figures 3A à 3D.

En référence à la Figure 3A, qui détaille l'étape de traitement 500, une fois que l'énergie de départ a été déterminée et qu'une apnée ou hypopnée a été détectée (étape 510), le système applique la stimulation avec cette énergie (étape 520). Si le système détecte un arrêt du trouble respiratoire (étape 530), il mémorise cette énergie comme efficace (étape 540). Dans le cas contraire, le système augmente la consigne d'énergie de stimulation d'un incrément donné (étape 560), puis déclenche une nouvelle stimulation (retour à l'étape 520) avec cette nouvelle consigne et continue la stimulation jusqu'à atteindre l'arrêt du trouble respiratoire. Entre les étapes 530 et 560 est par ailleurs prévue une étape 550 destinée à limiter l'énergie de stimulation appliquée à une énergie maximale admissible, qui peut être une valeur d'énergie absolue ou qui dépend des critères de sexe, d'âge, d'indice IMC et éventuellement d'autres critères, notamment le stade de sommeil du patient (sommeil à ondes lentes I, II, III, IV et sommeil paradoxal REM), l'énergie maximale étant alors fonction de ce stade de sommeil. Cette étape 550 peut être perfectionnée en prévoyant qu'en cas d'atteinte de la valeur d'énergie maximale admissible le système identifie, à l'aide des différents moyens capteurs 12, 14, 16 et 30, si l'épisode de trouble respiratoire est ou non un épisode grave : apnée trop longue, épisode désaturant, etc. (étape 570). Dans l'affirmative, l'énergie de stimulation est à nouveau augmentée d'un incrément (étape 560) pour donc dépasser la valeur maximale admissible, et un nouveau traitement de stimulation est effectué (étape 520), au risque certes de réveiller le patient, mais pour assurer une disparition du trouble.

La Figure 3B illustre en détail le processus 600 de mise à jour des deux valeurs d'énergie variables, à savoir la valeur de l'énergie considérée comme efficace pour faire disparaitre un trouble et l'énergie maximale admissible. Cette mise à jour est effectuée à la fin de chaque traitement. À l'étape 620, le système détermine si la valeur d'énergie efficace a été ou non incrémentée à l'étape 560 lors du traitement précédent. Dans l'affirmative, l'énergie de consigne de départ pour le prochain traitement est fixée comme étant la valeur d'énergie efficace après incrémentation(s) (étape 630). Dans la négative, l'énergie de consigne de départ est décré-mentée pour éviter une dérive du paramètre vers le haut (étape 640).

Pour ce qui concerne la valeur maximale admissible, le système détermine si la phase de traitement précédente 500 a provoqué ou non un réveil du patient (étape 650) et, dans l'affirmative, détermine à l'étape 660 si le niveau d'énergie qui a provoqué ce réveil est ou non inférieur à la valeur maximale admissible au moment du traitement : dans l'affirmative, le système fixe alors comme nouvelle valeur maximale admissible la valeur d'énergie qui provoqué le réveil (étape 670). Les moyens mis en oeuvre pour détecter le réveil peuvent varier selon les applications. Par exemple, cette détection peut se faire simplement à l'aide d'un activateur actionné par le patient lorsqu'il se réveille. Elle peut se faire automatiquement également à l'aide d'un capteur d'actimétrie ou grâce à l'étude de l'évolution de la ventilation comme est décrit dans le EP 1 317 943 A1 (ELA Medical).

Si en revanche, au test de l'étape 650 il est déterminé que la phase de traitement précédente 500 n'a pas provoqué de réveil, le système détermine alors si l'énergie de stimulation en fin de traitement (donc aboutissant à la disparition du trouble, sans réveil) était ou non supérieure à la valeur maximale admissible du moment (étape 680). Dans l'affirmative, le système fixe comme nouvelle valeur maximale admissible une valeur égale à la valeur précédente augmentée d'un incrément donné.

On comprend que ces deux ajustements permettent ainsi d'optimiser l'énergie de stimulation délivrée, en augmentant les chances d'aboutir à une disparition du trouble mais en minimisant en même temps les risques de réveil.

Dans cet exemple, cette optimisation se fait en continu au cours de la nuit après les épisodes traités. Dans une version simplifiée, il est aussi possible de n'optimiser ces valeurs qu'à l'issue d'une nuit complète, en se basant sur le taux de succès de la thérapie et le taux de réveil de la nuit passée pour application la nuit suivante.

La Figure 3C illustre en détail l'étape 200, à savoir la façon dont l'énergie de départ est déterminée lors du démarrage d'un traitement en fonction de la dernière énergie utilisée et de la valeur maximale admissible.

À l'étape 410, le système détermine si l'épisode de trouble du sommeil est le premier de la nuit, ce qui peut être déterminé facilement à l'aide d'un comptage incrémental des troubles. Dans l'affirmative, le système utilise pour le premier traitement l'énergie initiale déterminée en fonction des paramètres de sexe, d'âge et d'indice IMC (étape 420). Dans la négative, le système détermine (étape 430) si la valeur efficace utilisée lors d'un épisode précédent est inférieure à la valeur maximale admissible utilisée pour l'épisode précédent. Dans l'affirmative, la valeur d'énergie de départ est fixée comme étant la valeur d'énergie qui s'est avérée efficace lors de l'épisode précédent (étape 440). Dans la négative, le système utilise comme valeur d'énergie de départ la valeur maximale admissible utilisée lors de l'épisode précédent (étape 450).

La description qui précède est donnée à titre d'exemple pour une application ne considérant qu'un type de trouble respiratoire. Mais en fonction des types de troubles respiratoires, par exemple apnée, hypopnée centrale ou obstructive, limitation de débit, etc., on comprendra que les règles d'ajustement de l'énergie de stimulation peuvent être différentes. Notamment, le niveau d'énergie initialement délivré sera adapté. Ainsi la Figure 3 montre une étape optionnelle 700 prévue entre les étapes 100 et 200 et où le système détermine le type de trouble, de façon autonome à l'aide des moyens capteurs 12, 14, 16 ou préalablement à la mise en service, ou bien par saisie par le patient ou le personnel soignant du type de trouble constaté.

La Figure 3D détaille cette étape 700 : trois types de troubles peuvent être sélectivement diagnostiqués (étapes 710, 720, 730). En fonction du type de trouble détecté, le système choisit une certaine énergie de stimulation initiale (étapes 712, 722, 732), puis applique un traitement kinesthésique comme précédemment (étapes 714, 724, 734), et met à jour les valeurs d'énergie efficace admissible comme précédemment (étapes 716, 726, 736), pour chacun des types de troubles. La valeur d'énergie maximale admissible peut quant à elle être fixée de façon commune aux différents types de troubles (étape 740).

## Revendications

1. Un dispositif d'optimisation d'un traitement d'un trouble respiratoire chez un patient par stimulation kinesthésique, comprenant :
- des moyens (30, 18) de détection d'un trouble respiratoire ;
- des moyens de commande (18) aptes à produire des signaux de commande de stimulation kinesthésique en réponse à une détection d'un trouble respiratoire ;
- au moins un effecteur kinesthésique (20) apte à être appliqué sur un site cutané externe du patient, et comprenant un transducteur électromécanique vibrant apte à recevoir les signaux de commande et à délivrer une énergie de stimulation kinesthésique déterminée par lesdits signaux ;
- des moyens (12, 14, 16, 30) pour déterminer l'efficacité d'une stimulation, aptes à détecter une disparition dudit trouble respiratoire ; et
- des moyens (22) de détermination d'une énergie de stimulation, aptes à sélectionner une valeur d'énergie de départ et à faire varier la valeur d'énergie en fonction de l'efficacité d'une stimulation précédente, ladite valeur d'énergie étant plafonnée à une valeur maximale,
**caractérisé en ce qu'**il comprend en outre :
- une table mémorisée de valeurs d'énergie efficace minimale de départ, préalablement établie en fonction de données cliniques pour différentes combinaisons de sexe, d'âge, de taille et de poids et/ou en fonction d'un type de trouble respiratoire détecté ou paramétré,
et **en ce que** les moyens de détermination d'une énergie de stimulation sont aptes, préalablement à une première stimulation, à sélectionner une valeur initiale d'énergie efficace minimale de départ fonction de paramètres physiologiques du patient, à partir de ladite table mémorisée de valeurs d'énergie initiale.

2. Le système de la revendication 1, dans lequel les moyens de détermination d'une énergie de stimulation comprennent des moyens aptes, pour une stimulation faisant suite à une stimulation précédente, à déterminer la valeur d'énergie de départ en fonction d'une valeur d'énergie efficace ayant fait cesser le trouble respiratoire lors d'une stimulation précédente.

3. Le système de la revendication 1, **caractérisé en ce que** ladite valeur maximale est une valeur variable fonction d'au moins un facteur du groupe formé par : sexe, âge, indice IMC et stade de sommeil du patient, ainsi que le type de trouble respiratoire dont est affecté le patient.

4. Le système de la revendication 1, dans lequel les moyens de détermination d'une énergie de stimulation comprennent des moyens aptes à relever le niveau d'énergie de façon incrémentale à partir de la valeur d'énergie de départ, aussi longtemps qu'un trouble respiratoire intervenu ne disparait pas.

5. Le système de la revendication 4, dans lequel les moyens de détermination d'une énergie de stimulation comprennent des moyens aptes à comparer chaque valeur d'énergie incrémentée à la valeur maximale.

6. Le système de la revendication 5, dans lequel les moyens de détermination d'une énergie de stimulation comprennent des moyens aptes à fixer une valeur d'énergie supérieure à la valeur maximale admissible en cas de détection d'un épisode grave de trouble respiratoire.

7. Le système des revendications 3 et 6 prises en combinaison, dans lequel les moyens de détermination d'une énergie de stimulation comprennent des moyens aptes à relever la valeur maximale admissible d'énergie à la suite d'un dépassement de la valeur maximale admissible lors d'un trouble respiratoire précédent, sans réveil du patient.

## Patentansprüche

1. Vorrichtung zur Optimierung einer Behandlung einer Atemstörung bei einem Patienten durch kinästhetische Stimulation, umfassend:
- Mittel (30, 18) zum feststellen einer Atemstörung;
- Steuermittel (18), die in der Lage sind, kinästhetische Stimulationssteuersignale als Reaktion auf eine Feststellung einer Atemstörung zu erzeugen;
- mindestens einen kinästhetischen Effektor, der auf eine äußere Hautstelle des Patienten aufgebracht werden kann und einen vibrierenden elektromechanischen Wandler umfasst, der in der Lage ist, die Steuersignale zu empfangen und eine durch diese Signale bestimmte kinästhetische Stimulationsenergie zu liefern;
- Mittel (12, 14, 16, 30) zur Bestimmung der Effizienz einer Stimulation, die in der Lage ist, ein Verschwinden der Atemstörung zu detektieren; und
- Mittel (22) zur Bestimmung einer Stimulationsenergie, die in der Lage ist, einen Startenergiewert auszuwählen und den Energiewert entsprechend der Effizienz einer vorangehenden Stimulation zu variieren, wobei der Energiewert auf einen Maximalwert begrenzt ist und
**dadurch gekennzeichnet, dass** sie ferner umfaßt:
- eine gespeicherte Tabelle mit minimalen effektiven Start-Energiewerten, die zuvor nach klinischen Daten für verschiedene Kombinationen von Geschlecht, Alter, Größe und Gewicht und / oder nach einer Art von Atemwegserkrankung festgestellt oder festgelegt wurde,
und wobei die Mittel zur Bestimmung einer Stimulationsenergie, die in der Lage sind, vor einer ersten Stimulation, einen anfänglichen minimalen Start-Energiewert auf der Grundlage physiologischer Parameter des Patienten aus der gespeicherten Tabelle der anfänglichen Energiewerte auszuwählen.

2. System nach Anspruch 1, in welchem die Mittel zur Bestimmung einer Stimulationsenergie Mittel umfassen, die in der Lage sind, für eine Stimulation nach einer vorangehenden Stimulation, den Startenergiewert als Funktion eines effektiven Energiewertes, der die Atemstörung während eines vorherige Stimulation gestoppt hat festzustellen.

3. System nach Anspruch 1, **dadurch gekennzeichnet, dass** der Maximalwert ein variabler Wert ist, der auf mindestens einem Faktor der Gruppe basiert ist, die gebildet wird durch: Geschlecht, Alter, IMC-Index und Schlafstadium des Patienten sowie den Typ der Atemstörung, die den Patienten betrifft.

4. System nach Anspruch 1, in welchem die Mittel zur Bestimmung einer Stimulationsenergie Mittel umfassen, die in der Lage sind, den Energieniveau schrittweise von dem Startenergiewert zu erhöhen, solange eine Atemstörung, die geschehen ist, nicht verschwindet.

5. System nach Anspruch 4, in welchem die Mittel zum Bestimmung einer Stimulationsenergie Mittel umfassen, die in der Lage sind, jeden inkrementierten Energiewert mit dem Maximalwert zu vergleichen.

6. System nach Anspruch 5, in welchem die Mittel zur Bestimmung einer Stimulationsenergie Mittel umfassen, die in der Lage sind, einen Energiewert zu bestimmen, der höher ist als der maximal zulässige Wert im Falle des Nachweises einer schweren Episode der Atemstörung.

7. System nach Anspruch 3 und 6 in Kombination, in welchem die Mittel zur Bestimmung einer Stimulationsenergie Mittel umfassen, die in der Lage sind, den maximal zulässigen Energiewert zu erhöhen, indem der maximal zulässige Wert überschritten wird während einer früheren Atemstörung ohne den Patienten zu erwachen.

## Claims

1. A device for optimizing a treatment of a respiratory disorder in a patient by kinesthetic stimulation, comprising:
- means (30, 18) for detecting a respiratory disorder;
- control means (18) capable of producing kinesthetic stimulation control signals in response to a detection of a respiratory disorder;
- at least one kinesthetic effector capable of being applied to an external skin site of the patient and comprising a vibrating electromechanical transducer able to receive the control signals and to deliver a kinesthetic stimulation energy determined by said signals;
- means (12, 14, 16, 30) for determining the efficiency of a stimulation, capable of detecting a disappearance of said respiratory disorder; and
- means (22) for determining a stimulation energy, able to select a starting energy value and to vary the energy value according to the efficiency of a preceding stimulation, said energy value being capped at a maximum value, and
**characterized in that** it further comprises:
- a memorized table of minimum effective starting energy values, previously established according to clinical data for different combinations of sex, age, size and weight and / or according to a type of respiratory disorder detected or set, and **in that** the means for determining a stimulation energy are able, prior to a first stimulation, to select an initial minimum starting energy value based on physiological parameters of the patient, from said memorized table of initial energy values.

2. The system of claim 1, in which the means for determining a stimulation energy comprises means capable, for a stimulation following a preceding stimulation, to determine the starting energy value as a function of an effective energy value that has stopped the respiratory disorder during a previous stimulation.

3. The system of claim 1, **characterized in that** said maximum value is a variable value which is based on at least one factor of the group formed by: sex, age, IMC index and sleep stage of the patient, as well as the type of respiratory disorder affecting the patient.

4. The system of claim 1, in which the means for determining a stimulation energy comprises means able of raising the energy level incrementally from the starting energy value, as long as a respiratory disorder that has happened does not disappear.

5. The system of claim 4, in which the means for determining a stimulation energy comprises means able to compare each incremented energy value to the maximum value.

6. The system of claim 5, in which the means for determining a stimulation energy comprise means able to fix an energy value higher than the maximum admissible value in the event of the detection of a severe episode of respiratory disorder.

7. The system of claims 3 and 6 taken in combination, in which the means for determining a stimulation energy comprise means able of raising the maximum permissible energy value as a result of exceeding the maximum permissible value during a previous respiratory disorder, without the patient awakening.
